# EUROPÄISCHE PATENTSCHRIFT

(11) **EP 2 470 133 B1**
(45) Veröffentlichungstag und Bekanntmachung des Hinweises auf die Patenterteilung: **29.12.2021**
(21) Anmeldenummer: 10744603.1
(22) Anmeldetag: 23.08.2010
(51) Int. Cl.: A61F 13/00, A61F 13/534, A61F 13/02, A61F 13/06, A61F 13/04

(54) **WUNDPFLEGEARTIKEL MIT EINEM PLASTISCH VERFORM- ODER MODELLIERBAREN WIRKELEMENT**
WOUND CARE ARTICLE COMPRISING AN ACTIVE ELEMENT THAT CAN BE PLASTICALLY DEFORMED OR MOULDED
ARTICLE POUR LE SOIN DES PLAIES COMPRENANT UN ÉLÉMENT ACTIF EN PLASTIQUE POUVANT ÊTRE DÉFORMÉ OU MODELÉ

(30) Priorität: 24.08.2009 DE 102009038389; 28.09.2009 DE 102009043023
(43) Veröffentlichungstag der Anmeldung: 04.07.2012
(73) Patentinhaber: BSN medical GmbH, 22761 Hamburg (DE)
(72) Erfinder: Riesinger, Birgit, 48149 Münster (DE)
(74) Vertreter: FARAGO Patentanwälte
(86) Internationale Anmeldenummer: PCT/EP2010/062218
(87) Internationale Veröffentlichungsnummer: WO 2011/023650

(56) Entgegenhaltungen:
- EP-A1- 0 031 018
- EP-A1- 0 594 034
- EP-A1- 1 520 109
- WO-A1-2010/069592
- US-A- 3 596 657
- US-A- 5 429 633
- US-A1- 2004 241 215
- US-A1- 2005 137 539
- US-A1- 2007 078 366
- US-B1- 6 169 223

## Beschreibung

Die vorliegende Erfindung betrifft einen Wundpflegeartikel mit einem plastisch verform- oder modellierbaren Wirkelement.

Aus dem Stand der Technik, beispielsweise aus der DE10059439 der Anmelderin der vorliegenden Anmeldung, sind Wundpflegeartikel mit einem Wundexsudate absorbierenden Körper bekannt. Dabei weist letzterer einen im Wesentlichen flachen Materialabschnitt aus Absorptionsmaterial aus, bestehend aus einem aufsaugenden Vlies mit darin verteilten Superabsorber-Teilchen.

Diese Wundpflegeartikel haben sich in der Praxis außerordentlich bewährt, da sie in der Lage sind, Exsudate auch aus großer Tiefe aufzunehmen, und so die Wundheilung gerade bei chronischen und/oder ödembedingten Wunden erheblich zu verbessern.

Allerdings eignen sich diese Wundpflegeartikel ohne weiteres nur für relativ flache Wunden. Tiefgehende Wunden verlangen hingegen nach einem Wundfüllmaterial. Hierzu muss die Wunde also zunächst mit einem geeigneten Füller verfüllt werden, und anschließend wird dann der genannte Wundpflegeartikel aufgelegt. Dies erfordert in vielen Fällen ein umständliches Hantieren, beeinträchtigt u.U. das Aufnahmevermögen des Wundpflegeartikels und bringt Sepsisprobleme mit sich. Hinzu kommt, dass ein so hergestellter Verband leicht auseinanderfällt.

Überdies eignen sich solche Wundpflegeartikel nur für Wunden im Bereich einer relativ flachen Körpertopographie, so z.B. für die Auflage auf Beinen oder dem Thorax. In Bereichen komplexerer Körpertopographie (beispielweise an der Ferse oder am Kreuzbein) eignen sich solche Wundpflegeartikel nicht ohne weiteres für eine erfolgreiche Wundversorgung.

Aufgabe der vorliegenden Erkundung ist es, einen Wundpflegeartikel bereitzustellen, der sich sowohl für die Versorgung tiefer Wunden als auch für die Versorgung von Wunden in Bereichen komplexerer Körpertopographie eignet.

Diese Aufgabe wird mit den Merkmalen des vorgelegten Hauptanspruchs gelöst.

Erfindungsgemäß ist ein Wundpflegeartikel vorgesehen, aufweisend mindestens eine flächige Lage enthaltend ein absorbierendes Material, wobei der Wundpflegeartikel mindestens ein Wirkelement aufweist, welches ihm mindestens abschnittsweise eine plastische Verform- oder Modellierbarkeit verleiht.

Der Begriff "Plastische Verformbarkeit" beschreibt die Fähigkeit fester Stoffe, sich unter einer Krafteinwirkung irreversibel zu verformen und diese Form nach der Einwirkung beizubehalten.

Der erfindungsgemäße Wundpflegeartikel weist gegenüber dem bekannten Stand der Technik eine Reihe von Vorteilen auf:
So kann eine Pflegekraft die Form des erfindungsgemäßen Wundpflegeartikels der Topologie und Form der zu behandelnden Wunde anpassen. Bei einer tiefen Wunde kann der erfindungsgemäße Wundpflegeartikel durch plastische Verformung in eine zumindest abschnittsweise konvexe Form gebracht werden, sodass der Wundpflegeartikel die Wunde ausfüllt und über weite Flächen mit dem Wundgrund in Berührung kommt. Auf diese Weise werden auch tiefliegende Ödeme und Exsudatansammlungen erfasst, und der Wundheilungsprozess wird gefördert.

Ferner macht der erfindungsgemäße Wundpflegeartikel andere Wundfüller, wie Sie im Stand verwendet werden (siehe oben) verzichtbar.

Ferner werden so die superabsorbierenden Polymere ganz nah an den Wirkort gebracht - anders als bei den oben beschriebenen Wundfüllern, die in der Regel passiv sind, d.h. nicht mit aktiv wirkenden Agenzien ausgerüstet sind.

Dies ist besonders vorteilhaft, da superabsorbierende Polymere Proteine und Bakterien binden können, wie z.B. in der DE1020007054127 der Anmelderin der vorliegenden Erfindung gezeigt ist. Je näher sie an die Wunde gebracht werden, desto vorteilhafter wirkt sich diese Eigenschaft daher auf die Wundheilung aus.

Durch die plastische Verformbarkeit kann der Wundpflegeartikel aber auch in eine konkave Form gebracht werden, um z.B. um einen Körperteil wie einen Arm, ein Bein oder einen Finger herumgelegt zu werden.

Weiterhin sind natürlich sämtliche Kombinationen von konkaver und konvexer Verformung denkbar, um so den erfindungsgemäßen Wundpflegeartikel an alle denkbaren anatomischen Gegebenheiten voranzupassen.

Erfindungsgemäß ist dabei vorgesehen, dass das besagte Wirkelement eine Armierung aus einem plastisch verform- oder modellierbaren Material aufweist.

Unter dem Begriff "Armierung" ist im Folgenden eine stärkende Schicht gemeint, die dem Wundpflegeartikel seine plastische Verform- oder Modellierbarkeit verleiht, allerdings nicht an der Oberfläche des Wundpflegeartikels in Erscheinung tritt und folglich auch nicht mit der Wunde in Kontakt treten kann. Dies ist aus naheliegenden Gründen unerwünscht, da die Kontaktschicht zur Wunde weich, mehr oder minder elastisch und ggf. auch aufnahmefähig für Flüssigkeiten sein soll.

Erfindungsgemäß ist dabei vorgesehen, dass es sich bei dem plastisch verform- oder modellierbaren Material um ein Bandmaterial handelt. Letzteres besteht aus einem Kunststoff- oder Papierband, an dem seitlich zwei Drähte als Armierungen eingearbeitet sind.

In einer Ausführungsform, die nicht Teil der beanspruchten Erfindung ist, ist vorgesehen, dass das plastisch verform- oder modellierbare Material mindestens einen Werkstoff ausgewählt aus der Gruppe enthaltend Metall, Kunststoff, Hydrokolloidmaterial, Schaumstoff, Dentalabdruckmasse und/oder Biokunststoffe aufweist.

Als Metalle bei dieser nicht erfindungsgemäßen Ausführungsform kommen insbesondere Bänder, Plättchen oder Drähte aus Eisen, Gold, Silber, biokompatibler Metalllegierungen, wie Nirosta, und oder Formgedächtnismaterialien, wie z.B. NiTiNol, in Frage.

Als Kunststoff bei dieser nicht erfindungsgemäßen Ausführungsform kommen z.B. Bänder, Plättchen oder Drähte aus Polyisobutylen in Frage, es sind jedoch auch andere plastisch verform- oder modellierbare Kunststoffe geeignet.

Als Biokunststoffe bei dieser nicht erfindungsgemäßen Ausführungsform kommen insbesondere Polymilchsäure, Celluloid, Celluloseacetat, Polyhydroxyalkanoate, Polyhydroxybuttersäure, Hartgelatine, Chitosan und oder Galalith in Frage.

Der Begriff "Schaumstoff" bei dieser nicht erfindungsgemäßen Ausführungsform umfasst sowohl offen- also auch geschlossenzellige Schaumstoffe sowohl aus natürlichen Materialien (z.B. Stärke, Zellulose) als auch synthetischen Materialien (z.B. Polyurethan).

Der Begriff "Hydrokolloidmaterial" bei dieser nicht erfindungsgemäßen Ausführungsform bezieht sich auf plastisch verform- und modellierbare Materialien, wie sie z.B. im Bereich der Stomaversorgung zum Einsatz kommen. Hierbei handelt es sich beispielsweise um Gemische enthaltend Butylester von PVM/MA, Copolymer, Ethanol, Gelatine, Pektin, Polyacrylamid, Polysorbat, Silica, und/oder n-Butyl-Alkohol.

Der Begriff "Dentalabdruckmasse" bei dieser nicht erfindungsgemäßen Ausführungsform bezieht sich ebenfalls auf plastisch verform- und modellierbare Materialien, wie sie z.B. in der Dentalmedizin zum Einsatz kommen.

In einer Ausführungsform, die nicht Teil der beanspruchten Erfindung ist, ist dabei vorgesehen, dass die Armierung einen flächig angeordneten Materialverbund aufweist. Die Armierung aus einem flächig angeordneten Materialverbund ist dabei bevorzugt ausgewählt aus der Gruppe enthaltend
- ein Drahtgeflecht oder -gewirke
- eine plastisch verformbare Platte
- mehrere plastisch verformbare Plättchen
- eine plastisch verformbare Schaumstoffmatte
- eine plastisch verformbare Archimedische Spirale
- eine mindestens abschnittsweise plastisch verformbare Hülle
- eine Schüttung enthaltend Mikroperlen.

Weiterhin ist bevorzugt vorgesehen, dass das plastisch verform- oder modellierbare Draht- oder Bandmaterial beschichtet und/oder ummantelt ist.

Besagte Beschichtung und/oder Ummantelung kann beispielweise aus einem Lack, einem Latexmaterial, einem Silikonmaterial, einem Hydrokolloidmaterial (wie beispielweise Carboxymethylcellulose) und dergleichen erfolgen, und sie hat u.a. die Aufgabe, die Biokompatibilität des verform- oder modellierbaren Draht- oder Bandmaterials zu erhöhen. Weitere Aufgaben können eine Reduktion der thermischen Leitfähigkeit (wie sie insbesondere für metallisches Material zu beobachten ist), eine Reduktion der Oberflächenhärte des Draht- oder Bandmaterial, eine Verhinderung der Korrosion des Draht- oder Bandmaterials und dergleichen sein.

Die besagte Schüttung bei dieser nicht erfindungsgemäßen Ausführungsform enthaltend Mikroperlen ist beispielsweise aus Reisekissen bekannt, die durch diese Schüttung eine plastische Verformbarkeit erhalten. Die besagten Perlen bestehen bevorzugt aus Polystyrol, besonders bevorzugt können sie mit superabsorbierenden Polymeren durchmischt sein.

Besonders bevorzugt ist vorgesehen, dass das plastisch verform- oder modellierbare Material dergestalt ausgestattet ist, dass unter Einwirkung von Wärme und/oder Feuchtigkeit einen Teil seiner Festig- oder Steifigkeit verliert.

So kann z.B. ein plastisch verform- oder modellierbarer Kunststoff verwendet werden, der auch thermoplastische Eigenschaften hat, dergestalt, dass er bei Raumtemperatur (20 - 25 °C) relativ steif und plastisch verform- oder modellierbar ist, während er bei Temperaturen, wie sie bei am Patienten angelegten Zustand herrschen (32 - 38 °C), einen Teil seiner Steifigkeit verliert und so den Bewegungen, die der Patient ausübt, nachfolgt.

Ebenso kann ein plastisch verform- oder modellierbarer Kunststoff verwendet werden, der durch Kontakt mit wässrigen Flüssigkeiten, wie er z.B. bei Aufnahme von Exsudat in den Wundpflegeartikel erfolgt, seine Steifigkeit verliert und so den Bewegungen, die der Patient ausübt, nachfolgt. In beiden Fällen ist ein erheblicher Komfortgewinn für den Patienten die Folge, und es werden Probleme wie Hautreizungen und Wundscheuern vermieden. Die genannten Eigenschaften sind insbesondere bei Biokunststoffen zu verwirklichen.

Letztere können insbesondere auch so eingestellt sein, dass sie bei Kontakt mit wässrigen Flüssigkeiten nach und nach zerfallen.

Bevorzugt ist weiterhin vorgesehen, dass das absorbierende Material ein Vlies, bevorzugt ein Airlaid, aufweist.

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt. Der Begriff "Airlaid" bezeichnet einen speziellen Vliesstoff aus Zellstoff und Polyolefinfasern, in den ggf. superabsorbierende Polymere eingebettet sind.

Erfindungsgemäß ist dabei vorgesehen, dass das absorbierende Material superabsorbierende Polymere aufweist.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann.

Die superabsorbierenden Polymere können in dem erfindungsgemäßen Wundpflegeartikel in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Der Wundpflegeartikel kann bevorzugt einen im wesentlichen flachen Materialabschnitt aus Absorptionsmaterial aufweisen, der aus einem aufsaugenden Vlies mit darin verteilten superabsorbierenden Polymeren besteht. Diese können in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Dabei weist der Wundexsudate absorbierende Körper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und oder eine lose Füllung aus superabsorbierenden Polymeren. Besagte Airlaidmatte kann bevorzugt einen im wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Wundexsudate absorbierende Körper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion sachet" vertrieben wird.Der Wundexsudate absorbierende Körper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt.

Um besagten Kern herum kann eine Hülle angeordnet sein, die in Bereichen überlappend angeordnet ist, und der z.B. eine Klebenaht überdeckt bzw. Teil derselben ist.

Ebenso kann innerhalb der Hülle ein Abschnitt eines hydrophoben und/oder wasserabweisenden bzw. wasserundurchlässigen Materials vorgesehen sein, der Durchnässungs- oder Wäscheschutz fungiert.

Der Wundexsudate absorbierende Körper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor, sondern lediglich fixierte Benachbarungen beider Materialien. Die ggf. vorgesehenen mehreren Lagen können dabei in einer bevorzugten Ausgestaltung auch durch walzen, Pressen, Kalandrieren oder ähnliche Verfahren physisch miteinander verdichtet sein. Überdies kann der Körper sich wiederholende Musterungen oder Maserungen aufweisen, wie z.B. ein Karomuster, ein Stanzmuster oder dergleichen.

Bevorzugt ist weiterhin vorgesehen, dass der Wundpflegeartikel außerdem eine zumindest abschnittsweise flüssigkeitsdurchlässige Hülle aufweist.

Letztere umgibt den Materialabschnitt, bildet eine Barriere gegen feste Ausscheidungen bildet und ermöglicht den Durchtritt von anderen ausgetretenen Substanzen zu dem innerhalb der Hülle angeordneten Materialabschnitt aus Absorptionsmaterial. Die Hülle ist bevorzugt wenigstens teilweise von einer Naht abgeschlossen.

Besonders bevorzugt weist der Materialabschnitt in Draufsicht auf seine Flachseite eine Fläche (F1) auf, welche in seinem nicht benetzten Zustand um 3% bis 75% kleiner als die Fläche (F2) der flachgelegten Hülle ist und in der Hülle frei beweglich oder fixiert ist, wobei die Hülle ganzflächig Poren aufweist, welche jeweils kleiner als die unbenetzten superabsorbierenden Polymere sind.

Auf diese Weise ist gewährleistet, dass der Materialabschnitt bei Flüssigkeitsaufnahme in seinem Volumen zunehmen kann und nicht durch die Hülle eingeschränkt ist.

Besonders bevorzugt ist vorgesehen, dass die Hülle in Draufsicht auf seine Flachseite einen umlaufenden, über die Naht hinausragenden Oberstand aufweist und der Absorptionskörper frei von harten, scharfen Kanten und Ecken ist.

Die Poren oder Maschen der Hülle sind bevorzugt 0,05 mm bis 1,0 mm, vorzugsweise 0,20 mm bis 0,50 mm gross. Weiterhin kann bevorzugt vorgesehen sein, dass die Poren oder Maschen durch die Fäden- oder Faserabschnitte begrenzt sind, welche im Schnitt durch die Hülle etwa bogenförmig sind und mit ihren Bogen-Scheiteln nach aussen zeigen.

Die innere Lage des Absorptionskörpers weist bevorzugt ein flächenspezifisches Gewicht von wenigstens 300g/m² auf, wobei die Flächenmasse des darin gleichmassig verteilten Anteils der osmotisch wirksamen Substanzen wenigstens 100g/m² beträgt.

Besonders bevorzugt ist vorgesehen, dass die innere Lage zu mehr als 40 Gew.-% aus superabsorbierenden Polymeren besteht.

Die Umhüllung ist bevorzugt aus gewebten oder vliesartig zusammengesetzten Kunstfasern, wie Polypropylen- oder Polyethylenfasern, aber auch Baumwolle, Seide oder Viskose gebildet. Die Umhüllung besteht bevorzugt aus einem Gewebe oder Vlies, das wenigstens ein flächenspezifisches Gewicht von 20 g/m² aufweist.

Vorzugsweise besteht die Hülle aus hydrophoben Material, bzw. das Hüllenmaterial ist hydrophob ausgerüstet. Die hydrophoben Eigenschaften der Hülle verhindern das Verkleben mit der Wundoberfläche und tragen dazu bei, dass die Wundexsudat-Partikeln schneller ins Innere der Hülle gelangen können.

Dabei kann vorgesehen sein, dass die Hülle zumindest abschnittsweise ein elastisches Material aufweist, so z.B. Fasern aus Lycra oder Elasthan. Auch so ist gewährleistet, dass der Materialabschnitt bei Flüssigkeitsaufnahme in seinem Volumen zunehmen kann und nicht durch die Hülle eingeschränkt ist.

Die Umhüllung besteht bevorzugt aus einem aus einem Gewebe oder Vlies, das wenigstens ein flächenspezifisches Gewicht von 20 g/m² aufweist.

Wundauflagen der genannten Art sind beispielweise in der WO03094813, der WO2007051599 und der WO0152780 der Anmelderin der vorliegenden Erfindung offenbart..

Das Material der Hülle kann derart strukturiert sein, dass die Hülle eine raue Innenfläche und eine glatte Außenfläche aufweist. Vorzugsweise ist die raue Innenfläche der Hülle durch trichterförmige Perforationen gebildet, die sich jeweils in Richtung Innenfläche verjüngen und in eine freie Öffnungskante ("Auskragung") auslaufen. Diese raue Innenfläche wirkt den Verschiebungen des Inhaltes der Hülle entgegen, so dass auf eine Fixierung mit Klebepunkten verzichtet werden kann. Dementsprechend kann die glatte Außenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden, und ist z.B. aus der DE102006017194 der Anmelderin der vorliegenden Anmeldung bekannt.

Besonders bevorzugt ist dabei vorgesehen, dass das besagte dreidimensionale Hüllenmaterial auf das zuvor erwähnte Polypropylenvlies auflaminiert ist. Eine solche Ausgestaltung hat verbesserte Flüssigkeitsaufnahmeeigenschaften zur Folge.

Ebenso kann vorgesehen, sein, dass der Wundpflegeartikel auf seiner wundabgewandten Seite einen flüssigkeitsundurchlässigen Wäscheschutz ("Backsheet") aufweist. Weiterhin kann vorgesehen sein, dass die Hülle auf der wundzugewandten Seite mit einem physiologisch akzeptablen Kleber (beispielweise einem Hydrokolloidkleber) ausgerüstet ist.

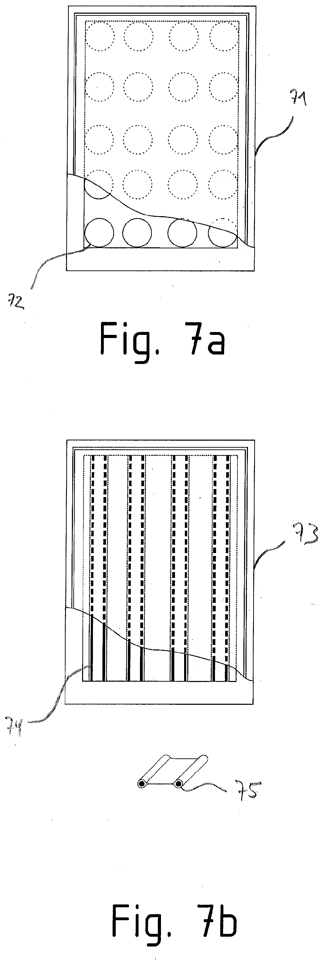

Die vorliegende Erkundung wird durch die im Folgenden gezeigten und diskutierten Figuren genauer erläutert. Dabei ist zu beachten, dass die Figuren nur beschreibenden Charakter haben und nicht dazu gedacht sind, die Erfindung in irgendeiner Form einzuschränken. Die Erfindung ist in den Abbildungen 7a und 7b dargestellt.

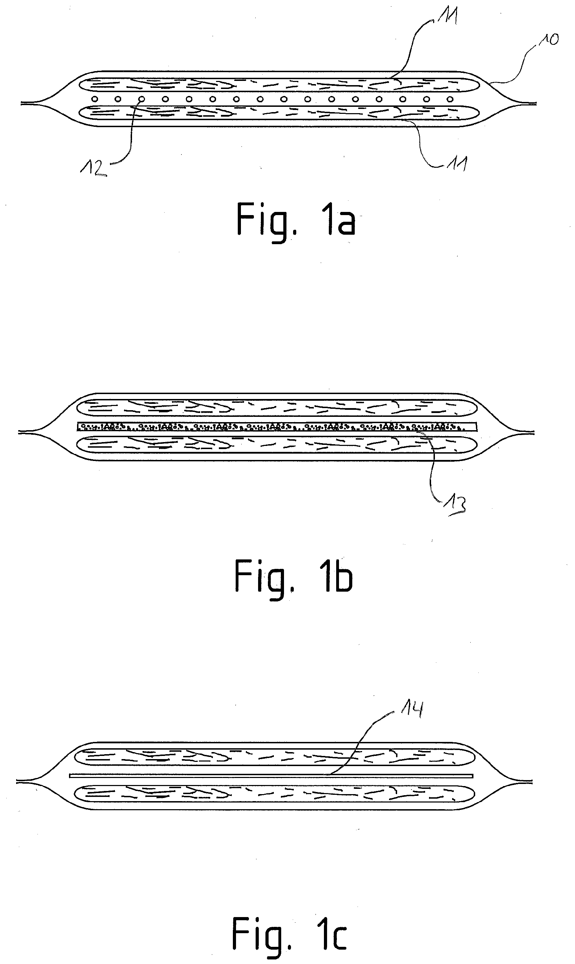

Figur la zeigt einen Wundpflegeartikel aufweisend zwei flächige Lagen 11 aufweisend ein absorbierendes Material sowie ein Wirkelement 12, welches dem Wundpflegeartikel eine plastische Verform- oder Modellierbarkeit verleiht. Das besagte Wirkelement liegt in Form eines Drahtgeflechts vor, das zwischen den beiden zwei-flächigen Lagen angeordnet ist und in Figur la im Anschnitt gezeigt ist. Figur 1b zeigt einen ähnlichen Wundpflegeartikel wie

Figur la mit dem Unterschied, dass es sich bei besagtem Wirkelement, welches dem Wundpflegeartikel eine plastische Verform- oder Modellierbarkeit verleiht, um eine plastisch verformbare Schaumstoffmatte 13 handelt.

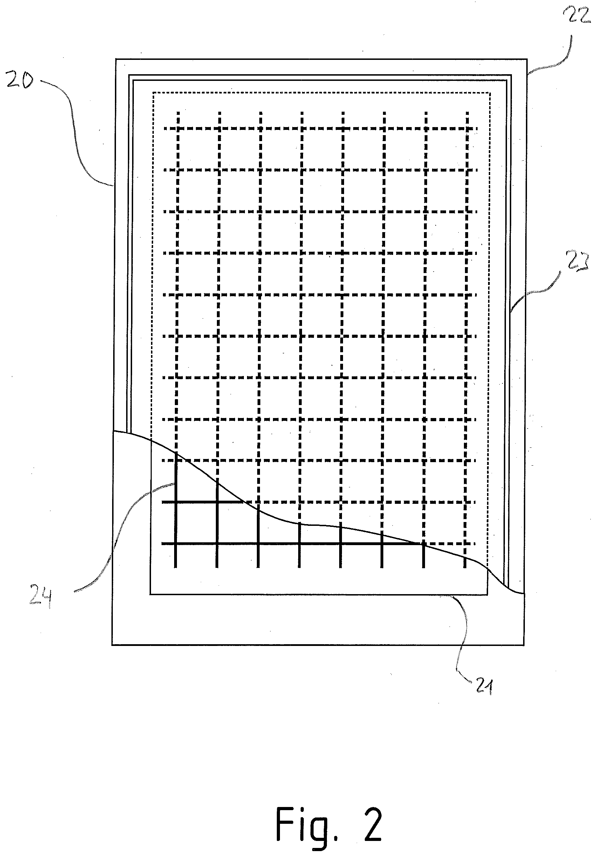

Figur le zeigt ebenfalls einen ähnlichen Wundpflegeartikel wie Figur la, mit dem Unterschied, dass es sich bei den Wirkelement, welches dem Wundpflegeartikel eine plastische Verform- oder Modellierbarkeit verleiht, um eine plastisch verformbare Platte 14 handelt. Figur 2 zeigt einen Wundpflegeartikel 20 in Aufsicht, wobei im unteren Bereich der Figur 2 der besagte Wundpflegeartikel im Anschnitt dargestellt ist. Der Wundpflegeartikel weist eine zwei-flächige Lage 21 auf, wovon nur die obere erkennbar ist. Besagte Lagen enthalten ein absorbierendes Material, bevorzugt einen Super-Absorber, und sind bevorzugt aus einem Vlies hergestellt. Figur 2 zeigt weiterhin, dass der Wundpflegeartikel eine Hülle 22 aufweist, die aus 2 Bahnen besteht, die über eine Naht 23 miteinander verbunden sind. Die Hülle besteht bevorzugt aus einem Polypropylen-Vlies, und bei der Naht 23 handelt sich bevorzugt um eine ultraschallfreie Naht.

Weiterhin ist in Figur 2 ein Wirkelement, welches dem Wundpflegeartikel eine plastische Verform- oder Modellierbarkeit verleiht, dargestellt. Hierbei handelt sich um ein Drahtgeflecht 24, das zwischen den beiden zwei-flächigen Lagen angeordnet ist. Das Drahtgeflecht 24 besteht z. B. aus einem feinen Nirosta-Draht; es sei angemerkt, dass die Maschenweite des besagten Drahtgeflechtes 24 in Figur 2 vergrößert dargestellt ist; bei dem tatsächlichen Produkt kann der Maschenabstand sehr viel geringer ausfallen.

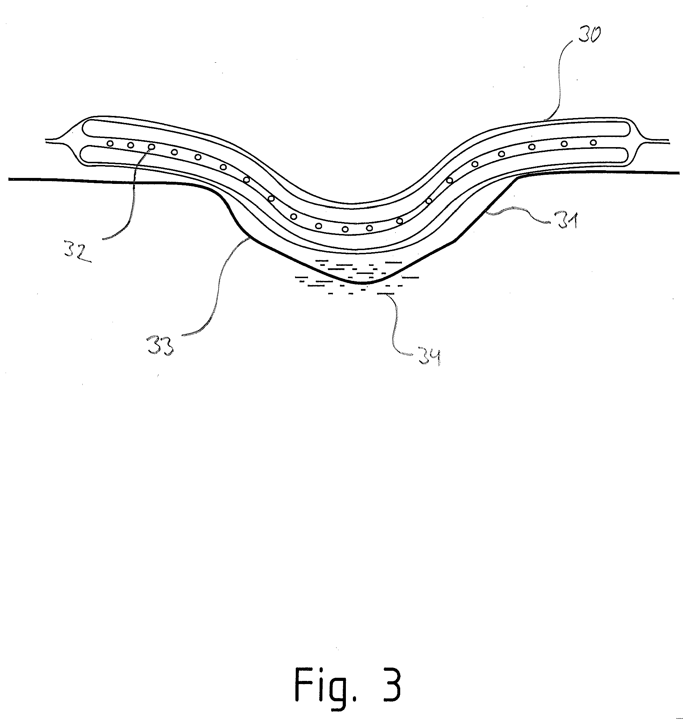

Figur 3 zeigt einen Wundpflegeartikel 30 in Anordnung auf einer tiefen Wunde 31. Es ist gut erkennbar, dass sich der Wundpflegeartikel aufgrund seiner plastischen Verformbarkeit, die durch das im Schnitt dargestellte Drahtgeflecht 32 bewirkt wird, gut an die Topologie und Form der zu behandelnden Wunde anpassen lässt. Dies kann z. B. eine Pflegekraft vor Anlegen des Wundpflegeartikels auf die Wunde bewerkstelligen. Auf dieses Weise kann z. B. bei einer tiefen Wunde der Wundpflegeartikel durch plastische Verformung in eine zumindest abschnittsweise konvexe Form gebracht werden, so dass der Wundpflegeartikel die Wunde ausfüllt und über weite Flächen mit dem Wundgrund 33 in Berührung kommt. Auf diese Weise werden tiefliegende Ödeme und Exsudatansammlungen 34 erfasst, und der Wundheilungsprozess wird gefördert.

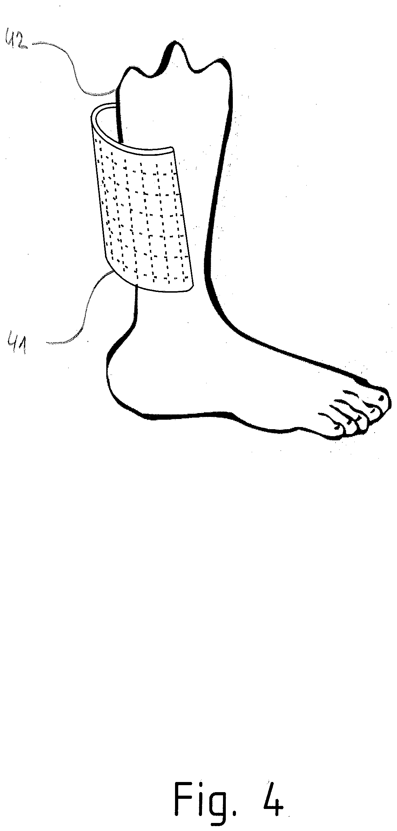

In Figur 4 ist dargestellt, dass der plastisch verformbare Wundpflegeartikel 41 nicht nur in eine konvexe Form gebracht werden kann, um z. B. tiefe Wunden auszufüllen, sondern auch in eine konkave Form, z. um den Wundpflegeartikel um ein Körperteil z. B. ein Bein 42 herum zu legen.

Auf diese Weise ist ein sicherer Halt des Wundpflegeartikels an besagtem Körperteil gewährleistet, und der Wundpflegeartikel ist so in der Lage, über seine gesamte Fläche mit der zu behandelnden Wunde in Kontakt zu stehen.

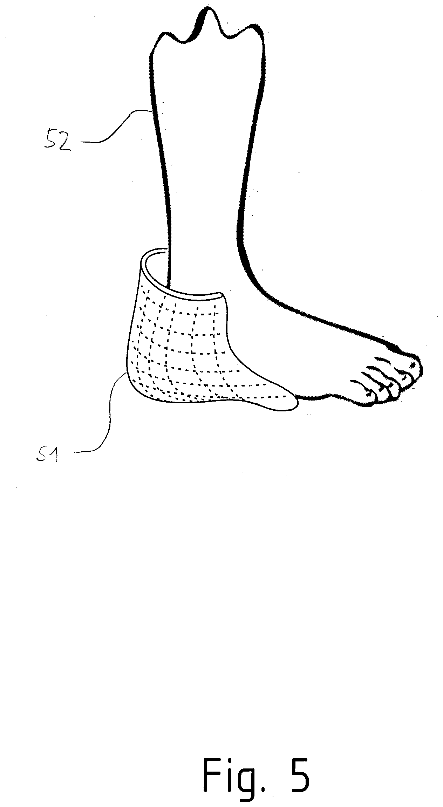

In Figur 5 ist überdies gezeigt, dass der Wundpflegeartikel aufgrund seiner plastischen Verform- oder Modellierbarkeit bereichsweise in eine konkave und bereichsweise auch in eine konvexe Form gebracht werden kann. So ist in Figur 5 gezeigt, dass der Wundpflegeartikel 51 z. B. um die Ferse 52 eines Patienten gelegt werden kann. Und hierbei handelt es sich um einen Körperteil mit einer sehr unregelmäßigen Topologie. Durch die plastische Verformbarkeit des Wundpflegeartikels wird jedoch unregelmäßigen Topologie Rechnung getragen und der Wundpflegeartikel kann dort einen sicheren Halt übernehmen und steht über seine gesamte Fläche mit der zu behandelnden Wunde in Verbindung.

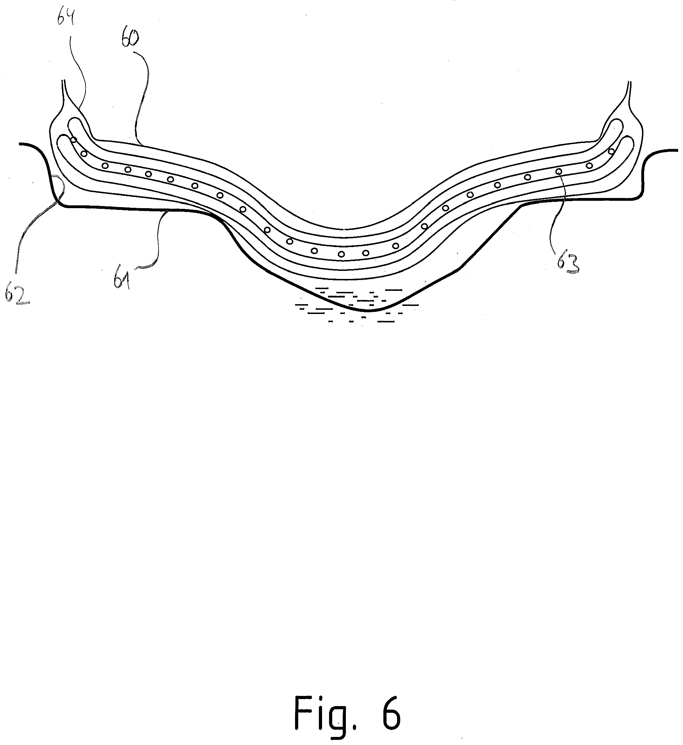

Figur 6 zeigt einen weiteren Wundpflegeartikel 60 in Anordnung auf einer Wunde 61 mit Wundrändern 62. Es ist gut erkennbar, dass sich der Wundpflegeartikel aufgrund seiner plastischen Verformbarkeit, die durch das im Schnitt dargestellte Drahtgeflecht 63 bewirkt wird, gut an die Topologie und Form der zu behandelnden Wunde anpassen lässt. Insbesondere an die Wundränder 62 kann der Wundpflegeartikel durch plastisches Hochbiegen seiner Ränder 64 hervorragend angepasst werden.

Figur 7a zeigt eine alternative Ausführungsform 71 des in Figur 2 gezeigten Wundpflegeartikels. Dabei liegen die Wirkelemente, welche dem Wundpflegeartikel eine plastische Verform- oder Modellierbarkeit verleihen, in Form von dünnen Metallplättchen 72. Diese sorgen, anders als in der Ausführungsform gemäß Figur 2, für eine abschnittsweise plastische Verform- oder Modellierbarkeit des betreffenden Wundpflegeartikels, beispielsweise um den Wundpflegeartikel an lokale Wundvertiefungen oder an tiefe Wundränder anzupassen.

Figur 7b zeigt die erfindungsgemäße Ausführungsform 73 des in Figur 2 gezeigten Wundpflegeartikels. Dabei liegen die Wirkelemente, welche dem Wundpflegeartikel eine plastische Verform- oder Modellierbarkeit verleihen, in Form von Plastik- oder Pappebändern 74 vor, an deren Rändern jeweils ein plastisch verformbarer Draht 75 eingearbeitet ist, wie in der Ausschnittsvergrößerung gezeigt.

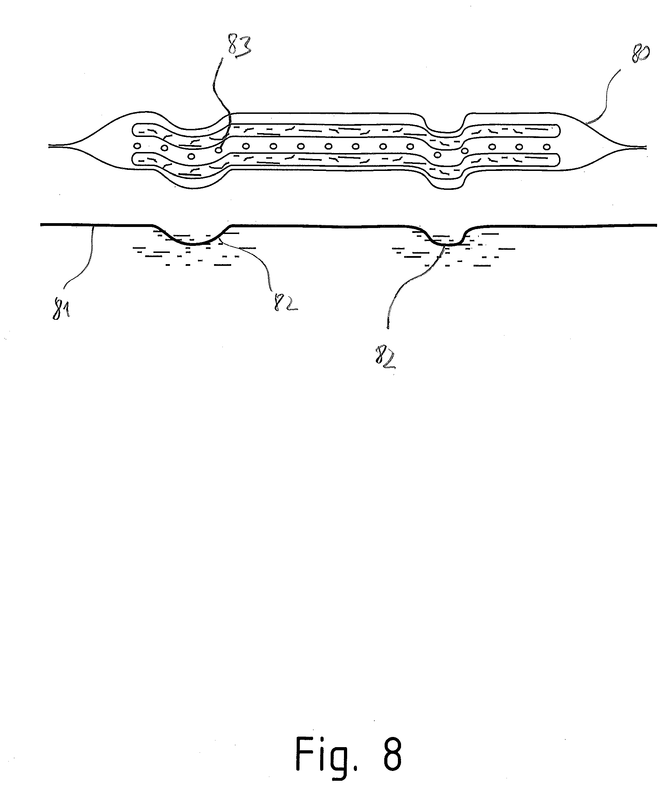

Figur 8 zeigt einen weiteren Wundpflegeartikel 80 in Anordnung auf einer Wunde 81 mit lokalen Vertiefungen 82. Es ist gut erkennbar, dass sich der Wundpflegeartikel aufgrund seiner plastischen Verformbarkeit, die durch das im Schnitt dargestellte Drahtgeflecht 83 bewirkt wird, gut an die Topologie und Form der zu behandelnden Wunde anpassen lässt. Insbesondere an die Vertiefungen 82 kann der Wundpflegeartikel hervorragend angepasst werden.

## Patentansprüche

1. Wundpflegeartikel (73), aufweisend mindestens eine flächige Lage (11) enthaltend ein absorbierendes Material, wobei das absorbierende Material superabsorbierende Polymere aufweist, und wobei der Wundpflegeartikel zusätzlich mindestens ein Wirkelement (12) aufweist, welches ihm mindestens abschnittsweise eine plastische Verform- oder Modellierbarkeit verleiht, **dadurch gekennzeichnet, dass** das Wirkelement eine Armierung in Form eines flächig angeordneten Materialverbundes aufweist, welches nicht an der Oberfläche des Wundpflegeartikels in Erscheinung tritt und das Wirkelement aus einem Kunststoff- oder Papierband (74) besteht, an dem seitlich zwei Drähte (75) als Armierungen eingearbeitet sind.

2. Wundpflegeartikel (73) gemäß einem der vorhergehenden Ansprüche, **dadurch gekennzeichnet, dass** das plastisch verform- oder modellierbare Draht- oder Bandmaterial (74, 75) beschichtet und/oder ummantelt ist.

3. Wundpflegeartikel (73) gemäß einem der vorhergehenden Ansprüche, **dadurch gekennzeichnet, dass** das plastisch verform- oder modellierbare Material dergestalt ausgestattet ist, dass es unter Einwirkung von Wärme und/oder Feuchtigkeit einen Teil seiner Festig- oder Steifigkeit verliert.

4. Wundpflegeartikel (73) gemäß einem der vorhergehenden Ansprüche, **dadurch gekennzeichnet, dass** das absorbierende Material ein Vlies, bevorzugt ein Airlaid, aufweist.

5. Wundpflegeartikel (73) gemäß einem der vorhergehenden Ansprüche, **dadurch gekennzeichnet, dass** der Wundpflegeartikel außerdem eine zumindest abschnittsweise flüssigkeitsdurchlässige Hülle (22) aufweist.

## Claims

1. Wound care article (73) comprising at least one planar layer (11) containing an absorbent material, the absorbent material containing super-absorbent polymers and the wound care article additionally containing at least one active element (12) which provides it, at least in portions, with plastic deformability or sculptability; **characterized in that** the active element contains a reinforcement in the form of a composite material arranged in planar form which does not appear on the surface of the wound care article, and **in that** the active element consists of a plastic or paper band (74) in which two wires (75) are integrated laterally as reinforcements.

2. Wound care article (73) according to the above Claim, **characterized in that** the plastically deformable or sculptable wire or band material (74, 75) is coated and/or sheathed.

3. Wound care article (73) according to one of the above Claims, **characterized in that** the plastically deformable or sculptable material is adapted to lose part of its rigidity or stiffness under the effect of heat and/or humidity.

4. Wound care article (73) according to one of the above Claims, **characterized in that** the absorbent material contains a non-woven, preferably an Airlaid.

5. Wound care article (73) according to one of the above Claims, **characterized in that** the wound care article additionally contains a covering (22) which is permeable to liquid, at least in portions.

## Revendications

1. Article de soin pour plaies (73) comportant au moins une couche à plat (11) contenant un matériau absorbant, sachant que le matériau absorbant comporte des polymères superabsorbants et sachant que l'article de soin pour plaie comporte en plus au moins un élément actif (12), lequel lui confère au moins par endroits une capacité de déformation et de malléabilité, **caractérisé en ce que** l'élément actif comporte une armature sous la forme d'un matériau composite disposé à plat, lequel n'apparaît pas à la surface de l'article de soin pour plaie et l'élément actif est composé d'une bande matière plastique ou de papier (74) sur laquelle sont intégrés sur le côté deux fils (75) en tant qu'armatures.

2. Article de soin pour plaies (73) selon la revendication précédente, **caractérisé en ce que** le matériau de fil ou de bande (74, 75) plastiquement déformable ou malléable est revêtu et/ou enrobé.

3. Article de soin pour plaies (73) selon l'une quelconque des revendications précédentes, **caractérisé en ce que** le matériau plastiquement déformable ou malléable est structuré de telle manière qu'il perd une partie de sa résistance ou rigidité sous l'effet de la chaleur et/ou de l'humidité.

4. Article de soin pour plaies (73) selon l'une quelconque des revendications précédentes, **caractérisé en ce que** le matériau absorbant comporte un matériau non-tissé, de préférence un matériau aérolique (type airlaid).

5. Article de soin pour plaies (73) selon l'une quelconque des revendications précédentes, **caractérisé en ce que** l'article de soin pour plaies comporte par ailleurs une enveloppe (22) perméable aux liquides au moins par endroits.
